# EUROPEAN PATENT SPECIFICATION

(11) **EP 4 381 475 B1**
(45) Date of publication and mention of the grant of the patent: **13.05.2026**
(21) Application number: 22852403.9
(22) Date of filing: 16.05.2022
(51) Int. Cl.: A61B 5/11, G06N 3/044, G06N 3/045, G06N 3/08, G06N 3/084, G06N 20/00, G06V 40/16, G10L 13/00, G10L 15/24, G06N 3/02, A61B 5/00

(54) **DETECTION OF SILENT SPEECH**
ERKENNUNG STILLER SPRACHE
DÉTECTION DE PAROLE SILENCIEUSE

(30) Priority: 04.08.2021 US 202163229091 P
(43) Date of publication of application: 12.06.2024
(73) Proprietor: Q (cue) Ltd., 5252213 Ramat Gan (IL)
(72) Inventor: MAIZELS, Aviad, 4730304 Ramat Hasharon (IL); BARLIYA, Avi, Tel Aviv (IL); KORNBLAU, Giora, Binyamina (IL); WEXLER, Yonatan, Jerusalem (IL)
(74) Representative: Beck Greener LLP
(86) International application number: PCT/IB2022/054527
(87) International publication number: WO 2023/012527

(56) References cited:
- WO-A1-2019/050881
- US-A1- 2010 131 268
- US-A1- 2016 027 441
- US-A1- 2019 189 145
- US-A1- 2020 370 879
- TAM�S GR�SZ, G�BOR GOSZTOLYA, L�SZL� T�TH, TAM�S G�BOR CSAP�, ALEXANDRA MARK�: "F0 Estimation for DNN-Based Ultrasound Silent Speech Interfaces", 2018 IEEE INTERNATIONAL CONFERENCE ON ACOUSTICS, SPEECH AND SIGNAL PROCESSING (ICASSP), IEEE, 15 April 2018 (2018-04-15), pages 291 - 295, XP033401010, DOI: 10.1109/ICASSP.2018.8461732
- GONZALEZ-LOPEZ JOSE A ET AL: "Silent Speech Interfaces for Speech Restoration: A Review", IEEE ACCESS, IEEE, USA, vol. 8, 23 September 2020 (2020-09-23), pages 177995 - 178021, XP011812908, DOI: 10.1109/ACCESS.2020.3026579

## Description

The present invention relates generally to physiological sensing, and particularly to methods and apparatus for sensing human speech.

The process of speech activates nerves and muscles in the chest, neck, and face. Thus, for example, electromyography (EMG) has been used to capture muscle impulses for purposes of speech sensing.

Secondary speckle patterns have been used for monitoring movement of skin on the human body. Secondary speckle typically occurs in diffuse reflections of a laser beam from a rough surface, such as the skin. By tracking both temporal and amplitude changes of secondary speckle produced by reflection from human skin when illuminated by a laser beam, investigators have measured blood pulse pressure and other vital signs. For example, U.S. Patent 10,398,314 describes a method for monitoring conditions of a subject's body using image data that is indicative of a sequence of speckle patterns generated by the body. US2020370879 discloses wearable devices, their configurations, and methods of operation that use self-mixing interferometry signals of a self-mixing interferometry sensor to recognize user inputs. WO2019050881 discloses a system that is able to detect silent, internal articulation of words by a human user, by measuring low-voltage electrical signals at electrodes positioned on a user's skin.

Embodiments of the present invention that are described hereinbelow provide novel methods and devices for sensing human speech.

In accordance with an aspect of the present invention, there is provided a sensing device as set out in the first of the appended independent claims. In accordance with another aspect of the present invention, there is provided a method for sensing as set out in the second of the appended independent claims. Features of various embodiments are set out in the appended dependent claims.

There is also disclosed herein examples of a sensing device, including a bracket configured to fit an ear of a user of the device and an optical sensing head held by the bracket in a location in proximity to a face of the user and configured to sense light reflected from the face and to output a signal in response to the detected light. Processing circuitry is configured to process the signal to generate a speech output.

In one example, the bracket includes an ear clip. Alternatively, the bracket includes a spectacle frame. In a disclosed example, the optical sensing head is configured to sense the light reflected from a cheek of the user.

The optical sensing head includes an emitter configured to direct coherent light toward the face and an array of sensors configured to sense a secondary speckle pattern due to reflection of the coherent light from the face. The emitter is configured to direct multiple beams of the coherent light toward different, respective locations on the face, and the array of sensors is configured to sense the secondary speckle pattern reflected from the locations. Additionally or alternatively, the locations illuminated by the beams and sensed by the array of sensors extend over an area of at least 1 cm². Further additionally or alternatively, the optical sensing head includes multiple emitters, which are configured to generate respective groups of the beams covering different, respective areas of the face, and the processing circuitry is configured to select and actuate a subset of the emitters without actuating all the emitters.

In a disclosed example, the processing circuitry is configured to detect changes in the sensed secondary speckle pattern and to generate the speech output responsively to the detected changes.

Alternatively or additionally, the processing circuitry is configured to operate the array of sensors at a first frame rate, to sense, responsively to the signal while operating at the first frame rate, a movement of the face, and to increase the frame rate responsively to the sensed movement to a second frame rate, greater than the first frame rate, for generating the speech output.

In a disclosed example, the processing circuitry is configured to generate the speech output responsively to changes in the signal output by the optical sensing head due to movements of a skin surface of the user without any utterance of sounds by the user.

Typically, the optical sensing head is held by the bracket in a position that is at least 5 mm away from a skin surface of the user.

In one example, the device includes one or more electrodes configured to contact a skin surface of the user, wherein the processing circuitry is configured to generate the speech output responsively to the electrical activity sensed by the one or more electrodes together with the signal output by the optical sensing head.

Additionally or alternatively, the device includes a microphone configured to sense sounds uttered by the user. In one example, the processing circuitry is configured to compare the signal output by the optical sensing head to the sounds sensed by the microphone in order to calibrate the optical sensing head. Additionally or alternatively, the processing circuitry is configured to change an operational state of the device responsively to sensing of the sounds uttered by the user.

In some examples, the device includes a communication interface, wherein the processing circuitry is configured to encode the signal for transmission over the communication interface to a processing device, which processes the encoded signals to generate the speech output. In a disclosed example, the communication interface includes a wireless interface.

Additionally or alternatively, the device includes a user control, which is connected to the bracket and configured to sense a gesture made by the user, wherein the processing circuitry is configured to change an operational state of the device responsively to the sensed gesture.

Further additionally or alternatively, the device includes a speaker configured to fit in the ear of the user, wherein the processing circuitry is configured to synthesize an audio signal corresponding to the speech output for playback by the speaker.

There is also disclosed herein examples of a method for sensing, which includes sensing a movement of skin on a face of a human subject in response to words articulated by the subject without vocalization of the words by the subject and without contacting the skin. Responsively to the sensed movement, a speech output is generated including the articulated words.

In some examples, sensing the movement includes sensing light reflected from the face of the subject. In the disclosed examples, sensing the light includes directing coherent light toward the skin and sensing a secondary speckle pattern due to reflection of the coherent light from the skin. In one example, directing the coherent light includes directing multiple beams of the coherent light toward different, respective locations on the face, and sensing the secondary speckle pattern reflected from each of the locations using an array of sensors.

In a disclosed example, generating the speech output includes synthesizing an audio signal corresponding to the speech output. Alternatively or additionally, generating the speech output includes transcribing the words articulated by the subject.

The present invention will be more fully understood from the following detailed description of the embodiments thereof, taken together with the drawings in which:
Fig. 1 is a schematic pictorial illustration of a system for speech sensing;
Fig. 2 is a schematic sectional view of an optical sensing head;
Fig. 3 is a schematic pictorial illustration of a speech sensing device;
Fig. 4 is a block diagram that schematically illustrates functional components of a system for speech sensing; and
Fig. 5 is a flow chart that schematically illustrates a method for speech sensing.

People communicate through their mobile telephones in nearly all locations and at all times. The widespread use of mobile telephones in public spaces creates a cacophony of noise and often raises privacy concerns, since conversations are easily overheard by passersby. At the same time, when one of the parties in a telephone conversation is in a noisy location, the other party or parties may have difficulty in understanding what they are hearing due to background noise. Text communications provide a solution to these problems, but text input to a mobile telephone is slow and interferes with the users' ability to see where they are going.

Embodiments of the present invention that are described herein address these problems using silent speech, enabling users to articulate words and sentences without actually vocalizing the words or uttering any sounds at all. The normal process of vocalization uses multiple groups of muscles and nerves, from the chest and abdomen, through the throat, and up through the mouth and face. To utter a given phoneme, motor neurons activate muscle groups in the face, larynx, and mouth in preparation for propulsion of air flow out of the lungs, and these muscles continue moving during speech to create words and sentences. Without this air flow, no sounds are emitted from the mouth. Silent speech occurs when the air flow from the lungs is absent, while the muscles in the face, larynx, and mouth continue to articulate the desired sounds.

Silent speech can arise as the result of neurological and muscular pathologies; but it can also occur intentionally, for example when we articulate words but do not wish to be heard by others. This articulation can occur even when we conceptualize spoken words without opening our mouths. The resulting activation of our facial muscles gives rise to minute movements of the skin surface. The inventors have found that by properly sensing and decoding these movements, it is possible to reconstruct reliably the actual sequence of words articulated by the user.

Thus, embodiments of the present invention that are described herein sense fine movements of the skin and subcutaneous nerves and muscles on a subject's face, occurring in response to words articulated by the subject with or without vocalization, and use the sensed movements in generating a speech output including the articulated words. These embodiments provide methods and devices for sensing these fine movements without contacting the skin, for example by sensing light reflected from the subject's face. They thus enable users to communicate with others or to record their own thoughts silently, in a manner that is substantially imperceptible to other parties. Devices and methods in accordance with these embodiments are also insensitive to ambient noise and can be used in substantially any environment, without requiring users to divert their view and attention from their surroundings.

Some embodiments of the present invention provide sensing devices having the form of common consumer items, such as a clip-on headphone or spectacles. In these embodiments, an optical sensing head is held in a location in proximity to the user's face by a bracket that fits in or over the user's ear. The optical sensing head senses light reflected from the face, for example by directing coherent light toward an area of the face, such as the cheek, and sensing changes in the secondary speckle pattern that arises due to reflection of the coherent light from the face. Processing circuitry in the device processes the signal output by the optical sensing head due to the reflected light to generate a corresponding speech output.

Alternatively, the principles of the present invention may be implemented without an ear clip or other bracket. For example, in an alternative embodiment, a silent speech sensing module, including a coherent light source and sensors, may be integrated into a mobile communication device, such as a smartphone. This integrated sensing module senses silent speech when the user holds the mobile communication device in a suitable location in proximity to the user's face.

The term "light," as used in the present description and in the claims, refers to electromagnetic radiation in any or all of the infrared, visible, and ultraviolet ranges.

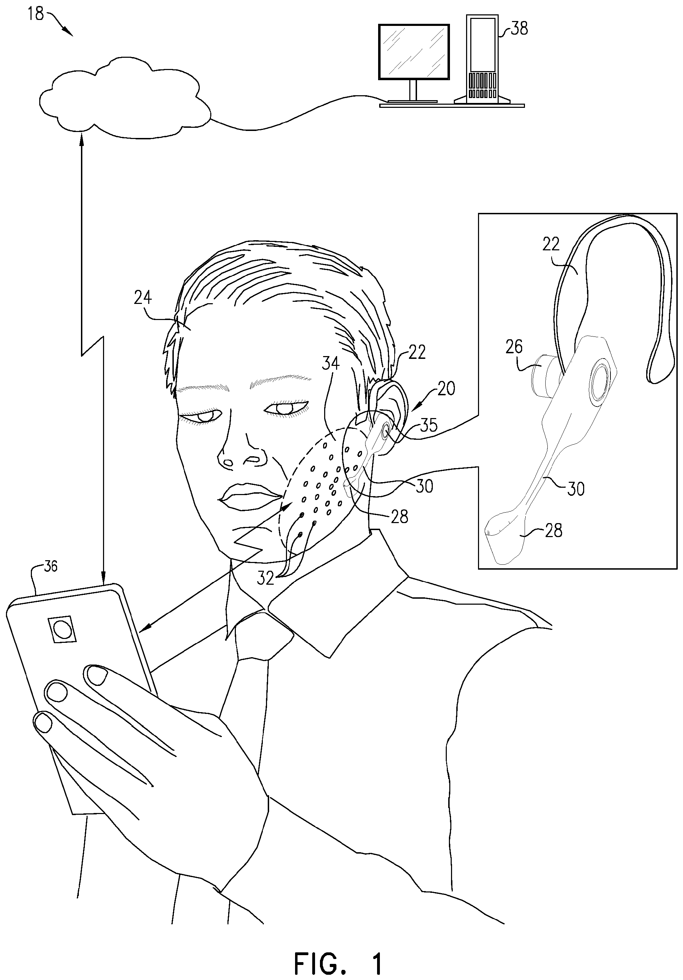

Fig. 1 is a schematic pictorial illustration of a system 18 for speech sensing, in accordance with an embodiment of the invention. System 18 is based on a sensing device 20, in which a bracket, in the form of an ear clip 22, fits over the ear of a user 24 of the device. An earphone 26 attached to ear clip 22 fits into the user's ear. An optical sensing head 28 is connected by an arm 30 to ear clip 22 and thus is held in a location in proximity to the user's face. In the pictured embodiment, device 20 has the form and appearance of a clip-on headphone, with the optical sensing head in place of (or in addition to) the microphone.

Optical sensing head 28 directs one or more beams of coherent light toward different, respective locations on the face of user 24, thus creating an array of spots 32 extending over an area 34 of the face (and specifically over the user's cheek). In the present embodiment, optical sensing head 28 does not contact the user's skin at all, but rather is held at a certain distance from the skin surface. Typically, this distance is at least 5 mm, and it may be even greater, for example at least 1 cm or even 2 cm or more from the skin surface. To enable sensing the motion of different parts of the facial muscles, the area 34 covered by spots 32 and sensed by optical sensing head 28 typically has an extent of at least 1 cm²; and larger areas, for example at least 2 cm² or even greater than 4 cm², can be advantageous.

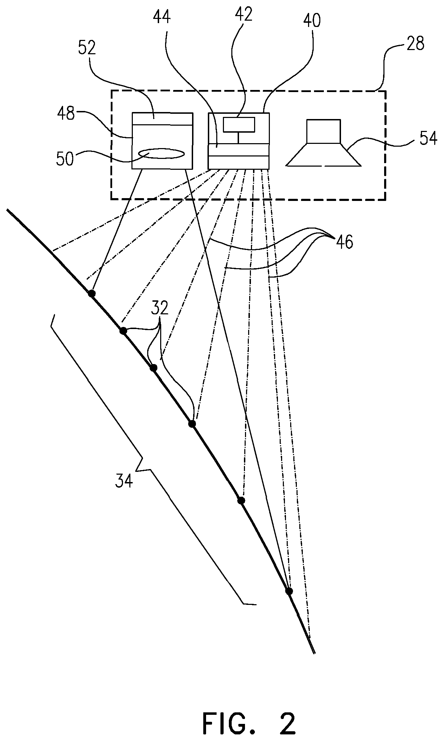

Optical sensing head 28 senses the coherent light that is reflected from spots 32 the face and outputs a signal in response to the detected light. Specifically, optical sensing head 28 senses the secondary speckle patterns that arise due to reflection of the coherent light from each of spots 32 within its field of view. To cover a sufficiently large area 34, this field of view typically has a wide angular extent, typically with an angular width of at least 60^{O}, or possibly 70^{O} or even 90^{O} or more. Within this field of view, device 20 may sense and process the signals due to the secondary speckle patterns of all of spots 32 or of only a certain subset of spots 32. For example, device 20 may select a subset of the spots that is found to give the largest amount of useful and reliable information with respect to the relevant movements of the skin surface of user 24. Details of the structure and operation of optical sensing head 28 are described hereinbelow with reference to Fig. 2.

Within system 18, processing circuitry processes the signal that is output by optical sensing head 28 to generate a speech output. As noted earlier, the processing circuitry is capable of sensing movements of the skin of user 22 and generating the speech output, even without vocalization of the speech or utterance of any other sounds by user 22. The speech output may take the form of a synthesized audio signal or a textual transcription, or both. The synthesized audio signal may be played back via the speaker in earphone 26 (and is useful in giving user 22 feedback with respect to the speech output). Additionally or alternatively, the synthesized audio signal may be transmitted over a network, for example via a communication link with a mobile communication device, such as a smartphone 36.

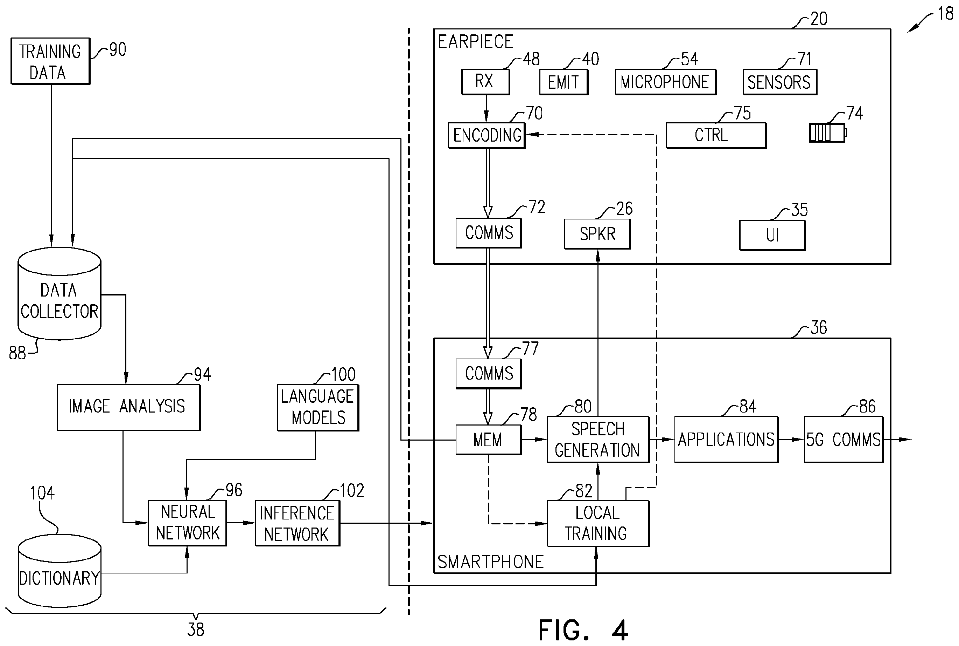

The functions of the processing circuitry in system 18 may be carried out entirely within device 20, or they may alternatively be distributed between device 20 and an external processor, such as a processor in smartphone 36 running suitable application software. For example, the processing circuitry within device 20 may digitize and encode the signals output by optical sensing head 28 and transmit the encoded signals over the communication link to smartphone 36. This communication link may be wired or wireless, for example using the Bluetooth^{™} wireless interface provided by the smartphone. The processor in smartphone 36 processes the encoded signal in order to generate the speech output. Smartphone 36 may also access a server 38 over a data network, such as the Internet, in order to upload data and download software updates, for example. Details of the design and operation of the processing circuitry are described hereinbelow with reference to Fig. 4.

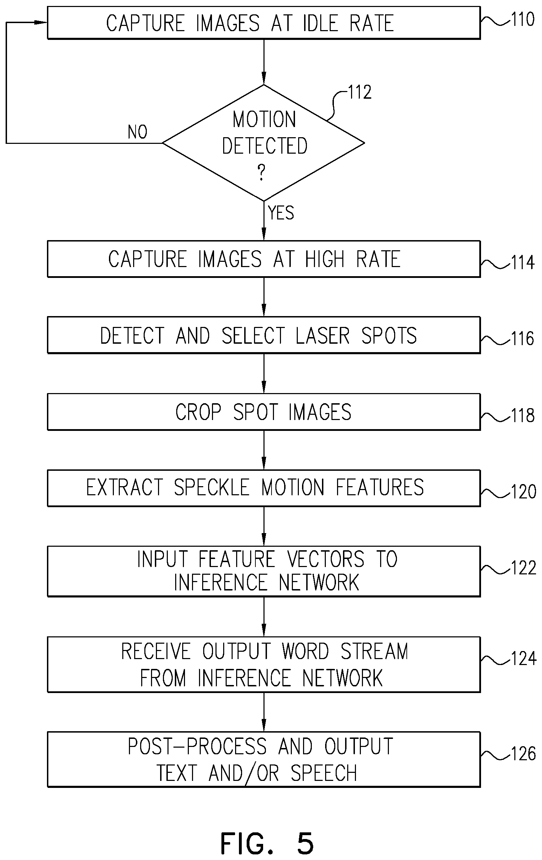

In the pictured embodiment, device 20 also comprises a user control 35, for example in the form of a push-button or proximity sensor, which is connected to ear clip 22. User control 35 senses gestures performed by user, such as pressing on user control 35 or otherwise bringing the user's finger or hand into proximity with the user control. In response to the appropriate user gesture, the processing circuitry changes the operational state of device 20. For example, user 24 may switch device 20 from an idle mode to an active mode in this fashion, and thus signal that the device should begin sensing and generating a speech output. This sort of switching is useful in conserving battery power in device 20. Alternatively or additionally, other means may be applied in controlling the operational state of device 20 and reducing unnecessary power consumption, for example as described below with reference to Fig. 5.

Fig. 2 is a schematic sectional view of optical sensing head 28 of device 20, showing components and functional details of the optical sensing head in accordance with an embodiment of the invention. Optical sensing head 28 comprises an emitter module 40 and a receiver module 48, along with an optional microphone 54.

Emitter module 40 comprises a light source, such as an infrared laser diode 42, which emits an input beam of coherent radiation. A beamsplitting element 44, such as a Damman grating or another suitable type of diffractive optical element (DOE), splits the input beam into multiple output beams 46, which form respective spots 32 at a matrix of locations extending over area 34. In one embodiment (not shown in the figures) emitter module 40 comprises multiple laser diodes or other emitters, which generate respective groups of the output beams 46, covering different respective sub-areas within area 34 of the user's face. In this case, the processing circuitry in device 20 may select and actuate only a subset of the emitters, without actuating all the emitters. For example, to reduce the power consumption of device 20, the processing circuitry may actuate only one emitter or a subset consisting of two or more emitters that illuminates the area on the user's face that has been found to give the most useful information for generating the desired speech output.

Receiver module 48 comprises an array 52 of optical sensors, for example, a CMOS image sensor, with objective optics 50 for imaging area 34 onto array 52. Because of the small dimensions of optical sensing head 28 and its proximity to the skin surface, receiver module 48 has a sufficiently wide field of view, as noted above, and views many of spots 32 at a high angle, far from the normal. Because of the roughness of the skin surface, the secondary speckle patterns at spots 32 can be detected at these high angles, as well.

Microphone 54 senses sounds uttered by user 24, enabling user 22 to use device 20 as a conventional headphone when desired. Additionally or alternatively, microphone 54 may be used in conjunction with the silent speech sensing capabilities of device 20. For example, microphone 54 may be used in a calibration procedure, in which optical sensing head 28 senses movement of the skin while user 22 utters certain phonemes or words. The processing circuitry may then compare the signal output by optical sensing head 28 to the sounds sensed by microphone 54 in order to calibrate the optical sensing head. This calibration may include prompting user 22 to shift the position of optical sensing head 28 in order to align the optical components in the desired position relative to the user's cheek.

In another embodiment, the audio signals output by microphone 54 can be used in changing the operational state of device 20. For example, the processing circuitry may generate the speech output only if microphone 54 does not detect vocalization of words by user 24. Other applications of the combination of optical and acoustic sensing that is provided by optical sensing head 28 with microphone 54 will be apparent to those skilled in the art after reading the present description and are considered to be within the present disclosure.

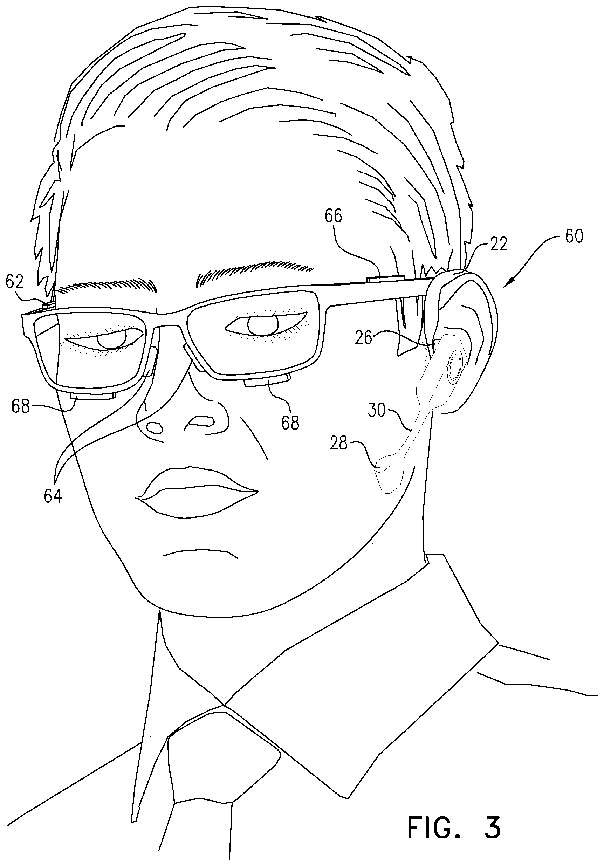

Fig. 3 is a schematic pictorial illustration of a speech sensing device 60, in accordance with another embodiment of the invention. In this embodiment, ear clip 22 is integrated with or otherwise attached to a spectacle frame 62. Nasal electrodes 64 and temporal electrodes 66 are attached to frame 62 and contact the user's skin surface. Electrodes 64 and 66 receive body surface electromyogram (sEMG) signals, which provide additional information regarding the activation of the user's facial muscles. The processing circuitry in device 60 uses the electrical activity sensed by electrodes 64 and 66 together with the output signal from optical sensing head 28 in generating the speech output from device 60.

Additionally or alternatively, device 60 includes one or more additional optical sensing heads 68, similar to optical sensing head 28, for sensing skin movements in other areas of the user's face. These additional optical sensing heads may be used together with or instead of optical sensing head 28.

Fig. 4 is a block diagram that schematically illustrates functional components of system 18 for speech sensing, in accordance with an embodiment of the invention. The pictured system is built around the components shown in Fig. 1, including sensing device 20, smartphone 36, and server 38. Alternatively, the functions illustrated in Fig. 4 and described below may be implemented and distributed differently among the components of the system. For example, some or all of the processing capabilities attributed to smartphone 36 may be implemented in sensing device; or the sensing capabilities of device 20 may be implemented in smartphone 36.

In the pictured example, as explained above, sensing device 20 comprises emitter module 40, receiver module 48, speaker 26, microphone 54, and user control (UI) 35. For the sake of completeness, sensing device 20 is shown in Fig. 4 as comprising other sensors 71, as well, such as electrodes and/or environmental sensors; but as noted earlier, sensing device 20 is capable of operation based solely on non-contact measurements made by the emitter and receiver modules.

Sensing device 20 comprises processing circuitry in the form of an encoder 70 and a controller 75. Encoder 70 comprises hardware processing logic, which may be hard-wired or programmable, and/or a digital signal processor, which extracts and encodes features of the output signal from receiver module 48. Sensing device 20 transmits the encoded signals via a communication interface 72, such as a Bluetooth interface, to a corresponding communication interface 77 in smartphone 36. A battery 74 provides operating power to the components of sensing device 20.

Controller 75 comprises a programmable microcontroller, for example, which sets the operating state and operational parameters of sensing device 20 based on inputs received from user control 35, receiver module 48, and smartphone 36 (via communication interface 72). Some aspects of this functionality are described below with reference to Fig. 5. In an alternative embodiment, controller 75 comprises a more powerful microprocessor and/or a processing array, which processes the features of the output signals from receiver module 48 locally within sensing device and generates a speech output, independently of smartphone 36.

In the present embodiment, however, the encoded output signals from sensing device 20 are received in a memory 78 of smartphone 36 and processed by a speech generation application 80 running on the processor in smartphone 36. Speech generation application 80 converts the features in the output signal to a sequence of words, in the form of text and/or an audio output signal. Communication interface 77 passes the audio output signal back to speaker 26 of sensing device 20 for playback to the user. The text and/or audio output from speech generation application 80 is also input to other applications 84, such as voice and/or text communication applications, as well as a recording application. The communication applications communicate over a cellular or Wi-Fi network, for example, via a data communication interface 86.

The operations of encoder 70 and speech generation application 80 are controlled by a local training interface 82. For example, interface 82 may indicate to encoder 70 which temporal and spectral features to extract from the signals output by receiver module 48 and may provide speech generation application 80 with coefficients of a neural network, which converts the features to words. In the present example, speech generation application 80 implements an inference network, which finds the sequence of words having the highest probability of corresponding to the encoded signal features received from sensing device 20. Local training interface 82 receives the coefficients of the inference network from server 38, which may also update the coefficients periodically.

To generate local training instructions 82, server 38 uses a data repository 88 containing speckle images and corresponding ground truth spoken words from a collection of training data 90. Repository 88 also receives training data collected from sensing devices 20 in the field. For example, the training data may comprise signals collected from sensing devices 20 while users articulate certain sounds and words (possibly including both silent and vocalized speech). This combination of general training data 90 with personal training data received from the user of each sensing device 20 enables server 38 to derive optimal inference network coefficients for each user.

Server 38 applies image analysis tools 94 to extract features from the speckle images in repository 88. These image features are input as training data to a neural network 96, together with a corresponding dictionary 104 of words and a language model 100, which defines both the phonetic structure and syntactical rules of the specific language used in the training data. Neural network 96 generates optimal coefficients for an inference network 102, which converts an input sequence of feature sets, which have been extracted from a corresponding sequence of speckle measurements, into corresponding phonemes and ultimately into an output sequence of words. Further details of the network architecture and training process are described in the above-mentioned provisional patent application. Server 38 downloads the coefficients of inference network 102 to smartphone 36 for used in speech generation application 80.

Fig. 5 is a flow chart that schematically illustrates a method for speech sensing, in accordance with an embodiment of the invention. This method is described, for the sake of convenience and clarity, with reference to the elements of system 18, as shown in Figs. 1 and 4 and described above. Alternatively, the principles of this method may be applied in other system configurations, for example using sensing device 60 (Fig. 3) or a sensing device that is integrated in a mobile communication device.

As long as user 24 is not speaking, sensing device 20 operates in a low-power idle mode in order to conserve power in battery 74, at an idling step 110. In this mode, controller 75 drives array 52 of sensors in receiver module 48 at a low frame rate, for example twenty frames/sec. Emitter module 40 may also operate at a reduced output power. While receiver module 48 operates at this low frame rate, controller 75 processes the images output by array 52 in order to detect a movement of the face that is indicative of speech, at a motion detection step 112. When such movement is detected, controller 75 instructs receiver module 48, as well as other components of sensing device 20 to increase the frame rate, for example to the range of 100-200 frames/sec, to enable detection of changes in the secondary speckle patterns that occur due to silent speech, at an active capture step 114. Alternatively or additionally, controller 75 may increase the frame rate and power up other components of sensing device 20 in response to other inputs, such as actuation of user control 35 or instructions received from smartphone 36.

The images captured by receiver module 48 typically contain a matrix of projected laser spots 32, as illustrated in Fig. 1. Encoder 70 detects the locations of the spots in the images, at a spot detection 116. The encoder may extract features from all the spots; but to conserve power and processing resources, it is desirable that the encoder select a subset of the spots. For example, local training interface 82 may indicate which subset of spots contains the greatest amount of information with respect to the user's speech, and encoder 70 may select the spots in this subset. Encoder 70 crops small windows from each image, with each such window containing one of the selected spots, at a cropping step 118.

Encoder 70 extracts features of speckle motion from each selected spot, at a feature extraction step 120. For example, encoder 70 may estimate the total energy in each speckle, based on the average intensity of the pixels in the corresponding window, and may measure the changes in energy of each speckle over time. Additionally or alternatively, encoder 70 may extract other temporal and/or spectral features of the speckles in the selected subset of spots. Encoder 70 conveys these features to speech generation application 80 (running on smartphone 36), which inputs vectors of the feature values to the inference network 102 that was downloaded from server 38, at a feature input step 122.

Based on the sequence of feature vectors that is input to the inference network over time, speech generation application 80 outputs a stream of words, which are concatenated together into sentences, at a speech output step 124. As noted earlier, the speech output is used to synthesize an audio signal, for playback via speaker 26. Other applications 84 running on smartphone 36 post-process the speech and/or audio signal to record the corresponding text and/or to transmit speech or text data over a network, at a post-processing step 126.

It will be appreciated that the embodiments described above are cited by way of example, and that combinations and subcombinations of the various features described hereinabove, as well as variations and modifications thereof which would occur to persons skilled in the art upon reading the foregoing description and which are not disclosed in the prior art are possible.

## Claims

1. A sensing device (20, 60), comprising:
a bracket (22) configured to fit an ear of a user (24) of the device;
an optical sensing head (28) held by the bracket in a location in proximity to a face of the user and configured to sense light reflected from the face and to output a signal in response to the detected light; and
processing circuitry (70, 75) configured to process the signal to generate a speech output;
**characterized in that** the optical sensing head comprises an emitter configured to direct coherent light toward the face and an array of sensors configured to sense a secondary speckle pattern due to reflection of the coherent light from the face.

2. The device according to claim 1, wherein the bracket comprises an ear clip or a spectacle frame.

3. The device according to claim 1, wherein the optical sensing head is configured to sense the light reflected from a cheek of the user.

4. The device according to claim 1, wherein the emitter is configured to direct multiple beams of the coherent light toward different, respective locations on the face, and the array of sensors is configured to sense the secondary speckle pattern reflected from the locations, wherein the locations illuminated by the beams and sensed by the array of sensors extend over a field of view having an angular width of at least 60° and an area of at least 1 cm².

5. The device according to claim 1, wherein the processing circuitry is configured to detect changes in the sensed secondary speckle pattern and to generate the speech output responsively to the detected changes.

6. The device according to claim 1, wherein the processing circuitry is configured to operate the array of sensors at a first frame rate, to sense, responsively to the signal while operating at the first frame rate, a movement of the face, and to increase the frame rate responsively to the sensed movement to a second frame rate, greater than the first frame rate, for generating the speech output.

7. The device according to claims 1-6, wherein the processing circuitry is configured to generate the speech output responsively to changes in the signal output by the optical sensing head due to movements of a skin surface of the user without any utterance of sounds by the user.

8. The device according to claims 1-6, wherein the optical sensing head is held by the bracket in a position that is at least 5 mm away from a skin surface of the user.

9. The device according to claims 1-6, and comprising one or more electrodes configured to contact a skin surface of the user, wherein the processing circuitry is configured to generate the speech output responsively to the electrical activity sensed by the one or more electrodes together with the signal output by the optical sensing head.

10. The device according to claims 1-6, and comprising a microphone configured to sense sounds uttered by the user.

11. The device according to claims 1-6, and comprising a wireless communication interface, wherein the processing circuitry is configured to encode the signal for transmission over the communication interface to a processing device, which processes the encoded signals to generate the speech output.

12. The device according to claims 1-6, and comprising a user control, which is connected to the bracket and configured to sense a gesture made by the user, wherein the processing circuitry is configured to change an operational state of the device responsively to the sensed gesture.

13. The device according to claims 1-6, and comprising a speaker configured to fit in the ear of the user, wherein the processing circuitry is configured to synthesize an audio signal corresponding to the speech output for playback by the speaker.

14. A method for sensing, comprising:
sensing a movement of skin on a face of a human subject in response to words articulated by the subject without vocalization of the words by the subject and without contacting the skin; and
responsively to the sensed movement, generating a speech output including the articulated words;
**characterized in that** sensing the movement comprises directing coherent light toward the skin and sensing a secondary speckle pattern due to reflection of the coherent light from the skin.

## Patentansprüche

1. Sensorvorrichtung (20, 60), umfassend:
eine Halterung (22), die so konfiguriert ist, dass sie an ein Ohr eines Benutzers (24) der Vorrichtung passt;
einen optischen Sensorkopf (28), der von der Halterung an einer Stelle in der Nähe eines Gesichts des Benutzers gehalten wird und so konfiguriert ist, dass er vom Gesicht reflektiertes Licht erfasst und ein Signal als Reaktion auf das detektierte Lichts ausgibt; und
eine Verarbeitungsschaltung (70, 75), die so konfiguriert ist, dass sie das Signal verarbeitet, um eine Sprachausgabe zu erzeugen;
**dadurch gekennzeichnet, dass** der optische Sensorkopf einen Emitter umfasst, der so konfiguriert ist, dass er kohärentes Licht auf das Gesicht richtet, und eine Anordnung von Sensoren, die so konfiguriert sind, dass sie ein sekundäres Speckle-Muster aufgrund der Reflexion des kohärenten Lichts vom Gesicht erfasst.

2. Vorrichtung nach Anspruch 1, wobei die Halterung einen Ohrclip oder ein Brillengestell umfasst.

3. Vorrichtung nach Anspruch 1, wobei der optische Sensorkopf so konfiguriert ist, dass er das von einer Wange des Benutzers reflektierte Licht erfasst.

4. Vorrichtung nach Anspruch 1, wobei der Emitter so konfiguriert ist, dass er mehrere Strahlen des kohärenten Lichts auf verschiedene, jeweilige Stellen auf dem Gesicht richtet, und die Anordnung der Sensoren konfiguriert ist, um das von den Stellen reflektierte sekundäre Speckle-Muster zu erfassen, wobei die Stellen, die von den Strahlen beleuchtet und von der Anordnung von Sensoren erfasst werden, sich über ein Sichtfeld mit einer Winkelbreite von mindestens 60° und einer Fläche von mindestens 1 cm² erstrecken.

5. Vorrichtung nach Anspruch 1, wobei die Verarbeitungsschaltung so konfiguriert ist, dass sie Änderungen in dem erfassten sekundären Speckle-Muster erkennt und die Sprachausgabe als Reaktion auf die erkannten Änderungen erzeugt.

6. Vorrichtung nach Anspruch 1, wobei die Verarbeitungsschaltung so konfiguriert ist, dass sie die Anordnung von Sensoren mit einer ersten Bildrate betreibt, um, als Reaktion auf das Signal, während des Betriebs mit der ersten Bildrate, eine Bewegung des Gesichts zu erfassen und die Bildrate, als Reaktion auf die erfasste Bewegung, auf eine zweite Bildrate zu erhöhen, die größer ist als die erste Bildrate, um die Sprachausgabe zu erzeugen.

7. Vorrichtung nach einem der Ansprüche 1 bis 6, wobei die Verarbeitungsschaltung so konfiguriert ist, dass sie die Sprachausgabe als Reaktion auf Änderungen des von dem optischen Sensorkopf ausgegebenen Signals aufgrund von Bewegungen einer Hautoberfläche des Benutzers erzeugt, ohne dass der Benutzer Laute von sich gibt.

8. Vorrichtung nach einem der Ansprüche 1 bis 6, wobei der optische Sensorkopf von der Halterung in einer Position gehalten wird, die mindestens 5 mm von einer Hautoberfläche des Benutzers entfernt ist.

9. Vorrichtung nach einem der Ansprüche 1 bis 6, die eine oder mehrere Elektroden umfasst, die so konfiguriert sind, dass sie eine Hautoberfläche des Benutzers kontaktieren, wobei die Verarbeitungsschaltung so konfiguriert ist, dass sie die Sprachausgabe als Reaktion auf die von der einen oder den mehreren Elektroden erfasste elektrische Aktivität zusammen mit dem von dem optischen Messkopf ausgegebenen Signal erzeugt.

10. Vorrichtung nach einem der Ansprüche 1 bis 6, und umfassend ein Mikrofon, das so konfiguriert ist, dass es die Laute erfasst, die der Benutzer von sich gibt.

11. Vorrichtung nach einem der Ansprüche 1 bis 6 und umfassend eine drahtlose Kommunikationsschnittstelle, wobei die Verarbeitungsschaltung so konfiguriert ist, dass sie das Signal für die Übertragung über die Kommunikationsschnittstelle an eine Verarbeitungsvorrichtung kodiert, die die kodierten Signale verarbeitet, um die Sprachausgabe zu erzeugen.

12. Vorrichtung nach einem der Ansprüche 1 bis 6 und umfassend eine Benutzersteuerung, die mit der Halterung verbunden und so konfiguriert ist, dass sie eine vom Benutzer ausgeführte Geste erfasst, wobei die Verarbeitungsschaltung konfiguriert ist, um einen Betriebszustand der Vorrichtung als Reaktion auf die erfasste Geste zu ändern.

13. Vorrichtung nach einem der Ansprüche 1 bis 6, und umfassend einen Lautsprecher, der so konfiguriert ist, dass er in das Ohr des Benutzers passt, wobei die Verarbeitungsschaltung so konfiguriert ist, dass sie ein Audiosignal synthetisiert, das der Sprachausgabe für die Wiedergabe durch den Lautsprecher entspricht.

14. Verfahren zum Erfassen, umfassend:
Erfassen einer Bewegung der Haut auf einem Gesicht eines menschlichen Subjekts als Reaktion auf Worte, die das Subjekt artikuliert, ohne dass das Subjekt die Worte vokalisiert und ohne die Haut zu berühren;
und
Erzeugen einer Sprachausgabe, die die artikulierten Wörter einschließt, als Reaktion auf die erfasste Bewegung;
**dadurch gekennzeichnet, dass** das Erfassen der Bewegung das Richten von kohärentem Licht auf die Haut und das Erfassen eines sekundären Speckle-Musters aufgrund der Reflexion des kohärenten Lichts von der Haut umfasst.

## Revendications

1. Dispositif de détection (20, 60) comprenant :
un support (22) configuré pour s'adapter à une oreille d'un utilisateur (24) du dispositif ;
une tête (28) de détection optique maintenue par le support dans un emplacement à proximité du visage de l'utilisateur et configurée pour détecter la lumière réfléchie par le visage et pour délivrer en sortie un signal en réponse à la lumière détectée ; et
des circuits de traitement (70, 75) configurés pour traiter le signal pour générer une sortie vocale ;
**caractérisé en ce que** la tête de détection optique comprend un émetteur configuré pour diriger de la lumière cohérente vers le visage, et un réseau de capteurs configuré pour détecter un motif de chatoiement secondaire dû à la réflexion de la lumière cohérente par le visage.

2. Dispositif selon la revendication 1, dans lequel le support comprend un clip d'oreille ou une monture de lunettes.

3. Dispositif selon la revendication 1, dans lequel la tête de détection optique est configurée pour détecter la lumière réfléchie par une joue de l'utilisateur.

4. Dispositif selon la revendication 1, dans lequel l'émetteur est configuré pour diriger plusieurs faisceaux de la lumière cohérente vers différents emplacements respectifs sur le visage, et le réseau de capteurs est configuré pour détecter le motif de chatoiement secondaire réfléchi par les emplacements, dans lequel les emplacements éclairés par les faisceaux et détectés par le réseau de capteurs s'étendent sur un champ de vision présentant une largeur angulaire d'au moins 60° et une surface au moins de 1 cm².

5. Dispositif selon la revendication 1, dans lequel les circuits de traitement sont configurés pour détecter des modifications dans le motif de chatoiement secondaire détecté et pour générer la sortie vocale en réponse aux modifications détectées.

6. Dispositif selon la revendication 1, dans lequel les circuits de traitement sont configurés pour exploiter le réseau de capteurs à une première fréquence d'images, pour détecter, en réponse au signal tout en fonctionnant à la première fréquence d'images, un mouvement du visage, et pour augmenter la fréquence d'images en réponse au mouvement détecté jusqu'à une seconde fréquence d'images, supérieure à la première fréquence d'images, à des fins de génération de la sortie vocale.

7. Dispositif selon les revendications 1 à 6, dans lequel les circuits de traitement sont configurés pour générer la sortie vocale en réponse à des modifications dans le signal délivré en sortie par la tête de détection optique dus à des mouvements de la surface cutanée de l'utilisateur sans que l'utilisateur émette le moindre son.

8. Dispositif selon les revendications 1 à 6, dans lequel la tête de détection optique est maintenue par le support dans une position qui est située à au moins 5 mm de la surface cutanée de l'utilisateur.

9. Dispositif selon les revendications 1 à 6, et comprenant une ou plusieurs électrodes configurées pour entrer en contact avec la surface cutanée de l'utilisateur, dans lequel les circuits de traitement sont configurés pour générer la sortie vocale en réponse à l'activité électrique détectée par les une ou plusieurs électrodes conjointement au signal délivré en sortie par la tête de détection optique.

10. Dispositif selon les revendications 1 à 6, et comprenant un microphone configuré pour détecter les sons émis par l'utilisateur.

11. Dispositif selon les revendications 1 à 6, et comprenant une interface de communication sans fil, dans lequel les circuits de traitement sont configurés pour coder le signal à des fins de transmission via l'interface de communication vers un dispositif de traitement, qui traite les signaux codés pour générer la sortie vocale.

12. Dispositif selon les revendications 1 à 6, et comprenant une commande utilisateur qui est reliée au support et configurée pour détecter un geste effectué par l'utilisateur, dans lequel les circuits de traitement sont configurés pour modifier un état de fonctionnement du dispositif en réponse au geste détecté.

13. Dispositif selon les revendications 1 à 6, et comprenant un haut-parleur configuré pour s'adapter à l'oreille de l'utilisateur, dans lequel les circuits de traitement sont configurés pour synthétiser un signal audio correspondant à la sortie vocale à des fins de reproduction par le haut-parleur.

14. Procédé de détection comprenant :
la détection d'un mouvement de la peau sur le visage d'un sujet humain en réponse à des mots articulés par le sujet, sans que les mots soient vocalisés par le sujet et sans entrer en contact avec la peau ; et
en réponse au mouvement détecté, la génération d'une sortie vocale comprenant les mots articulés ;
**caractérisé en ce que** la détection du mouvement comprend le fait de diriger une lumière cohérente vers la peau et de détecter un motif de chatoiement secondaire dû à la réflexion de la lumière cohérente par la peau.
